# EUROPEAN PATENT APPLICATION

(11) **EP 3 409 297 A1**
(43) Date of publication of application: **05.12.2018**
(21) Application number: 17192192.7
(22) Date of filing: 20.09.2017
(51) Int. Cl.: A61K 51/10, A61P 35/00, A61K 103/40, A61K 103/00, C22B 3/42, C22B 30/06

(54) **THE OPTIMAL 225ACTINIUM--213BISMUTH GENERATOR FOR ALPHA-PARTICLE RADIOIMMUNOTHERAPY**

(30) Priority: 30.05.2017 WO PCT/EP2017/063064
(71) Applicant: AlfaRim Medial Holding B.V., 6891 CJ Rozendaal (NL)
(72) Inventor: GEERLINGS, Maurits Willem, 6881 LD Velp (NL)
(74) Representative: De Vries & Metman

(57) **Abstract**

The present invention is directed to a method for the preparation of a radiolabled conjugate composition comprising Bismuth-213 loaded targeting moieties that have been diluted with cold targeting moieties in a predetermined ratio of amount of cold targeting moieties to amount of Bismuth-213 loaded targeting moieties. Said ratio is determined by determining the amount T of target moieties per diseased cell to be treated, and determining the amount Z of Bismuth-213 atoms needed to kill a diseased cell, and contacting targeting moieties with Bismuth-213 atoms in a ratio of T over Z, to form a conjugate composition with a predetermined ratio of cold targeting moieties and Bismuth-213 loaded targeting moieties.

The invention is further directed to a conjugate that can be prepared with the method according to the invention, a pharmaceutical formulation comprising the conjugate according to the invention.

## Description

### FIELD OF THE INVENTION

The present invention relates to the field of site-directed therapy. More precisely the invention relates to the field of α-particle radio immuno therapy (α-RIT) and provides a method for the production of radioconjugates and an apparatus for radioimmunotherapy. The method, conjugates and apparatus can be practicalized without the need for radioactive shielding and/or airtight facilities. Without these restrictions the invention provides a simple and efficient means of radioimmunotherapy at the bedside of the patient.

### BACKGROUND OF THE INVENTION

Nowadays there are a number of methods of site-directed therapy which have been suggested to eliminate unwanted cells or other biological matter e.g. vascular occlusions, or infectious organisms from the body of a mammalian subject.
There are many fields of therapy in which such methods may be applied. The most important ones include, without limitation, immune diseases (either auto immune diseases or acquired immune diseases), cancer and viral or microbial infections.

Since 1990 there are about 15 patents covering the major issues involved with realizing the chemistry and equipment requirements to enable α-RIT on a regular basis in hospitals. It is striking that these patents, without exception, fail to address two different aspects needed to enable two key requirements for successfull α-RIT:
- To produce a therapeutical product that inherently assures a homogeneous repartition of the drug to the cancer cells, single or in clusters, throughout the body of the patient, and
- To operate the system in such a way that the maximum possible amount of daughter isotope in a decay cascade of isotopes between the source isotope and the therapeutically active product isotope in the generator will be harvested in order to enable a final therapeutic product cost that proves economically feasible.

It is the objective of this patent to present viable solutions to these two problems.

Site directed therapy is a method whereby a cytotoxic compound is delivered to the immediate vicinity of the target cell or infectious organism. This is usually done by coupling a targeting moiety to the cytotoxic compound.

This targeting moiety recognizes a structure in, on or near the target. Known targeting moieties include, but are not limited to, antibodies, more specifically monoclonal antibodies and more preferably human monoclonal antibodies, nucleic acids, receptor-directed ligands and the like. These targeting moieties recognize target moieties that are near or present at the target such as antigens or receptor ligands.

Cytotoxic compounds can be for instance drugs, such as adromycin, toxins such as ricin A and radioisotopes.
The cytotoxic compound after being coupled onto the targeting moiety will be the immunotherapeutical drug. It usually is administered to the patients blood stream via an infusion liquid. Once in the blood stream the targeting moiety of this product acts as kind of a military cruise missile carrying the cytotoxic compound as its warhead to the target.

Depending on their nature (emitting α-or β/γ rays), radioisotopes can be used not only for therapy, but also for diagnostics by identifying the site(s) of the target(s). Therapies with targeting moïeties are widely known and, with β-emitting isotopes nowdays widely applied.
Since 15 years a steadily growing interest is developing for α-particle radioimmunotherapy because of the huge potential advantages of α-particles over β-rays: The mass of α-particles is 7000 x greater than of P's, the kinetic energy at their emission for α's (5 - 8 MeV) is 30 x higher than of P's, the track length (along which the kinetic energy of the particles is dissipated) in biological tissue of α's at 50 - 80 µm compared with ≈ 500 mm for β's. Biological cell killing means causing a sufficient degree of DNA damage: Shooting with α-particles at DNA is like playing american billiards, shooting with a billiard ball on a cluster of billiard balls, compared with shooting with a ping-pong ball at a cluster of billiard balls in case of β's.

EP 0585986 discloses a method to produce conjugates of an antibody and ²¹²Bi. US 6403771 decribes the use of actinium or one of its daugthers in radioimmunotherapy. To this end a radioimmunoconjugate is prepared comprising a radionuclide that emits alpha particles, a chelating agent and an antibody specific for tumor associated antigens. The conjugates compositions described in these publications however are not optimally designed for effective treatment without creating too much damage to non-target sites, i.e. healthy tissue. The amount of Bismuth-213 needed per patient with the method described in US 6403771 is so high that it does not provide an economically feasible treatment. Further, with the method described it is not ensured that all bismuth-213 isotopes formed were bound to antibodies. To our knowledge none of the prior art solves these issues. Below a list of patent publications and other publications concerning radioimmunitherapy is given.

US 4001387,US 4296785, US 4305922, US 4454106, US 4472509, US 4663129, US 4732864, US 4816397, US 4828991, US 4833329, US 4894364, US 4923985, US 5038046, US 5246691, US 5254328, US 5292868, US 5355394, US 5428154, US 5443816, US 5641471, EP 0306168, WO 90/15625.

Macklis et al. Radioimmunotherapy with alpha-particle-emitting immunoconjugates. Science (1988), vol. 240, No. 4855, pp. 1024-1026.
- Rotmensch et al. Estimates of dose to intraperitoneal micrometastases from alpha and beta emitters in radioimmunotherapy. Gynecologic Oncology (1990), vol. 38, No. 3, pp. 478-485.
- D.S. Wilbur, "Potential Use of a .alpha.-emitting Radionucleotides in the Treatment of Cancer", Antibody, Immunoconjugates and Radiopharmaceuticals, 4(1): 85-97 (1991).
- T. Mitsugashira et al., "Preparation of Traces for Actinium, Thorium, Proactinium and Uranium", SPEY, Min. Educ. Sci. & Cult., Tokyo, 9: 111-116 (1984).
- S. Suzuki, "Solution Chemistry of Light Actinude Elements", Japan--US Seminar on Torium fuel reactors--Proceedings, Nara, Japan, Oct. 18-22, 1982 (Tokyo, 1985), pp. 137-143.
- S. Mirzadeh et al., "Radiometal Labeling of Immunoproteins: Covalent Linkage of 2-(-4Isothiocyanatobenzyl)di-ethylenetriaminepentaacetic Acid Ligends to Immunoglobulin", Bioconjugate Chem., 1: 59-65 (1990).
- Zwierzina et al., Stem Cells, 11: 144 (1993).
- Kozak et al., Tibtech, 4(10): 259 (1986).
- Goldenberg, Arch. Pathol. Lab Med., 112: 580 (1988)..
- M. Chatterjee et al., Cancer Immunol. Immunopathol., 38: 75-82 (1994).
- Osband, Immunol. Today. 11(6): 193-195 (1990)..
- Curti, Crit. Rev. Oncol. Hematol., 14: 29-39 (1993).
- Jain, Scientific American 271: (1): 58-65 (1994).
- D.R. Fischer, "Alpha-Particle Emitters in Medicine", Proceedings of a symposium held at Loews L'Enfant Plaza Hotel, Washington, DC, Sep. 21 and 22, 1989, pp. 194-214.
- M.R. McDevitt et al., "Alpha Particle Emitting Bismuth-213 Cyclohexylbenzyl DTPA Constructs of Monoclonal Antibodies for Therapy of Cancer", Cancer Res., 1998.
- M. Brechbiel and O.A. Gansow, Synthesis of C-Functionalized trans-Cyclohexyldiethylenetriaminepenta-acetic Acids for Labelling of Monoclonal Antibodies with the Bismuth-212 .alpha.-Particle Emitter, J. Chem. Soc. Perkin Trans., pp. 1173-1178 (1992).
- Scheinberg et al., "Targeting in Erythroleukemic Mice ... ", Monoclonal Antibodies Drug. Dev., Process Jacob Abel Symp. for Drug Dev., pp. 159-171 (1982).
- "Astatine-211: It's Possible Applications in Cancer Therapy", Brown, International Journal of Radiation Applications and Instrumentation, Part A, vol. 37, No. 8, pp. 789-798 (1986).
- "Preparation of .sup.224RA for Therapy of Ankylosing Spondylitis", Delikan Health, Physics, vol. 35, No. 1, pp. 21-24 (1978).
- Lambrecht, "Radionuclide Generators", Radiochimica Acts 34, 9-24 (1983), R. Oldenbourg Verlag, Munich, p. 19.
- Spitsyn & Mikheev, "Generators for the Production of Short-Lived Radioisotopes, " Atomic Energy Review, 9(4): 787 et seq. (1971), International Atomic Energy Commission, Vienna, pp. 820-823.
- Riechmann et al., "Reshaping human antibodies for therapy", Nature, 332:323-327 (1988), Macmillan Magazines, London.
- DeNardo et al., "Fractionated Radioimmunotherapy of 8-Cell Malignancies with .sup.131I-Lym-1", Cancer Res. (Suppl), 50: 1014s-1016s (1990).

### SUMMARY OF THE INVENTION

The present invention is directed to a method for the preparation of a radiolabled conjugate composition for treating diseased cells comprising targeting moieties and Bismuth-213 atoms wherein:
- The conjugate composition comprising Bismuth-213 loaded targeting moieties that have been diluted with cold targeting moieties in a predetermined ratio of amount of cold targeting moieties to amount of Bismuth-213 loaded targeting moieties,
- Said ratio is determined by:
   a. Determining the amount T of target moieties per diseased cell to be treated, and
   b. Determining the amount Z of Bismuth-213 atoms needed to kill a diseased cell, and
   c. Contacting targeting moieties with Bismuth-213 atoms in a ratio of T over Z,
to form a conjugate composition with a predetermined ratio of cold targeting moieties and Bismuth-213 loaded targeting moieties.

The invention is further directed to a method wherein step c is performed by guiding a stream of eluent comprising targeting moieties over an appropriate medium that is loaded with Actinium-225 for a period of time so that any Bismuth-213 formed is coupled to a targeting moieties. Said stream of eluent may be guided over the medium with a speed that is set to obtain a composition with the predetermined ratio in amount of cold targeting moieties and Bismuth-213 loaded targeting moieties.

In another embodiment of the invention the targeting moiety is a monoclonal antibody, or a fragment or a derivative thereof, which may be a human or humanized antibody, or a fragment or a derivative thereof.
In one embodiment the monoclonal antibody is directed to a complementary antibody of a tumour associated antigen.

The Bismuth-213 may be eluted using HCl, HI or a mixture thereof as eluent. The coupling of the targeting moiety and Bismuth-213 may be conducted by means of a chelating agent. In an embodiment of the invention targeting moiety is present in the eluent. In a preferred embodiment part of the amount of targeting moieties is added to the radiolabeled conjugate as cold targeting moiety.

The invention is further directed to a conjugate composition of a targeting moiety and a radioisotope which has been produced by a method according to the invention, a pharmaceutical formulation.

### BRIEF DESCRIPTION OF THE DRAWINGS

The above-described aspects will hereafter be more explained with further details and benefits with reference to the drawing showing an embodiment of the invention by way of example.

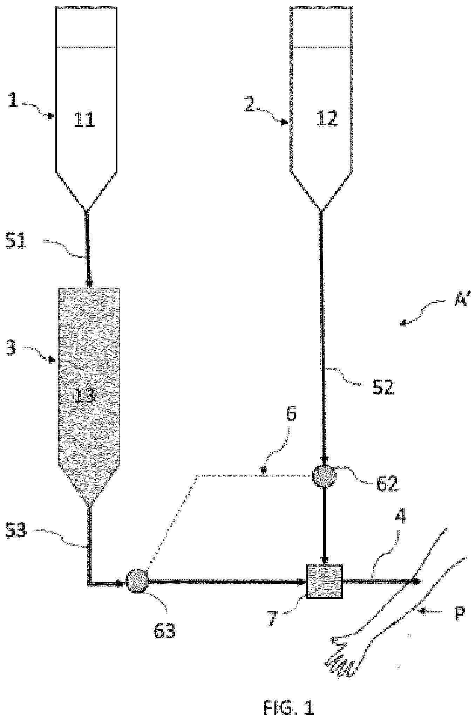

Fig. 1 schematically indicates another embodiment of an apparatus which typically may be used in practizing the invention as described here above.

### DETAILED DESCRIPTION OF THE INVENTION

The present invention aims at solving the above mention problems. To this end the invention provides a method for the preparation of a radiolabled conjugate composition for treating diseased cells comprising targeting moieties and Bismuth-213 atoms wherein:
- The conjugate composition comprising Bismuth-213 loaded targeting moieties that have been diluted with cold targeting moieties in a predetermined ratio of amount of cold targeting moieties to amount of Bismuth-213 loaded targeting moieties,
- Said ratio is determined by:
   a. Determining the amount T of target moieties per diseased cell to be treated, and
   b. Determining the amount Z of Bismuth-213 atoms needed to kill a diseased cell, and
   c. Contacting targeting moieties with Bismuth-213 atoms in a ratio of T over Z,
to form a conjugate composition with a predetermined ratio of cold targeting moieties and Bismuth-213 loaded targeting moieties.

The invention is based on the notion that diseased cells, such as for instance cancer cells, usually have very large amounts of tumor associated antigens on their surface. When using the complementary antibodies as targeting moiety, a very selective binding with the diseased cells occurs. We combined this with the knowledge that the short-range cell-killing effect of α-particles is enormous: a 1 mm diameter tumor, comprising about 600.000 cancer cells, needs about 6 α-particles of 6 Mev per cell to deliver a dose of 600 rad, causing a 99.9% cell-killing ratio and that specifically because of the stochastic nature of the hit-and-kill mechanism (using the example above for illustration) only 6 alpha particles need to arrive at the tumor to destroy the cancer cell. We found that it is possible to only load part of the targeting moieties and still ensure killing of the tumor cell. When only part of the targeting moieties are loaded also a homogenous distribution is ensured of the radiolabled targeting moieties over the diseased cells. Since no tumor-associated antigens are present at healthy cells, virually none of the targeting moiety will bind to the healthy tissue and cause damage to healthy tissue.

The present invention provides a method for the preparation of a radiolabled conjugate composition wherein the desired dilution of loaded targeting moieties with cold targeting moieties is optimally set. This is done by contacting the targeting moieties with the bismuth-213 atoms in a predetermined ratio.

### The amount of target moieties per diseased cell (T)

Said ratio is determined by first determining the amount T of target moieties per diseased cell. For a lot of cancer cell types the amount of cancer-associated antigens is known and usually varies from 10⁴ to 10⁶. If for a certain cancer cell type the amounts of antigens is not known it can easily be determined by measuring the amount of targeting moiety bonding to diseased cells. These methods are known in the art and need no further elucidation here. For instance for Acute Myeloid Leukemia (AML) it is known that the number of antigenic sites of a cancer cell's surface is greater than 10.000. The error in measuring the amount of target moieties per diseased cell ranges from 25 to over 50 %. Since the actual killing rate will also be dependent on the morphology of the cancer cells, the cancer load of the patient, and the binding time of the targeting moiety to its target moiety, even with this error, the predetermined ratio calculated herewith will provide a suitable guidance for the dilution needed in the conjugate composition.

### The amount of Bismuth-213 atoms needed to kill a diseased cell (Z)

Subsequently the amount Z of Bismuth-213 atoms needed to kill a diseased cell has to be determined. This amount is dependent from the type of cell and its form. In the case of AML cancer cells which usually have spherical shapes, it can be calculated that the conservative number of alpha particles needed to statistically kill such as cell is about 10. This is also the case for other cancer cells with approximately the same size and form. In the case of more irregularly shaped cancer cells the number of alpha atoms needed per cell may slightly differ, but usualy varies from 4 to 15 alpha particles. This type of calulations are known in the art and need no further elucidation here.

### Contacting targeting moieties with Bismuth-213 atoms in a ratio of T over Z

Subsequently targeting moieties are contacted with Bismuth-213 atoms in a ratio of T over Z. As an example in the case of the treatment of the AML-patients at Memorial Sloan-Kettering Cancer Center as described above, only 10 in 10.000 of the targeting moieties need to be bound to Bismuth-213 atoms. In that case a homogeneous distribution of radiolabeled targeting moiety is guaranteed over the total amount of diseased cells in the body of the patient. In addition to that, since a large overdose of targeting moieties is presented to the Bismuth-213 atoms, any bismuth-213 atom released from the actinium-225 is captured by a targeting moiety and the binding rate of the Bismuth to the targeting moiety will also be enhanced because of said overdose. This is important, because non-bound Bismuth-213 injected into a patient, will be disastrous. Therefore, with prior art conjugate compositions, the composition had to be analyzed for non-bound isotopes prior to injection to the patient. Because of the large overload of targeting moieties, any bismuth-213 formed will be caught and bound to the targeting moiety and no additional check is needed. Furthermore, an optimal use of the Actinium-225 and Bismuth-213 isotope is made. Since these isotopes are scarce and very expensive, the reduction of the amount of isotopes needed for a successful treatment will render the treatment less expensive and within reach to be used on commercial scale.
Step c of the method according to the invention may be performed by guiding a stream of eluent comprising targeting moieties over an appropriate medium that is loaded with Actinium-225 for a period of time so that any Bismuth-213 formed is coupled to a targeting moiety. Said stream of eluent may be guided over the medium with a speed that is set to obtain a composition with the predetermined ratio in amount of cold targeting moieties and Bismuth-213 loaded targeting moieties. Any deviations of the eluent speed may be adjusted over time to end up with the predetermined ratio.

A suitable way to load the Actinium-225 on an appropriate medium is to load the Actinium-225 on an ion-exchange column so that the Bismuth-213 when formed can be eluted by washing the substrate. Such a ion exchange column or other appropriate substrate filled with the actinium-225 can be placed at or near the bedside. Resins that are used in ion-exchange columns are commercially available and need no further elucidation here.

During elution, the Bismuth-213 is coupled to the targeting moiety and (optionally together with an infusion solution) the conjugate composition can be administered to the patient. This can all be done in a continuous mode with an apparatus according to the invention as shown in Fig. 1 or in an intermittent mode by using ordinary laboratory glassware. Suitable eluents for eluting the Bismuth-213 from the ion-exchange column are HCl, HI, and mixtures thereof.

Preferably the targeting moiety has been previously added to the eluting solution so that the coupling can start taking place in the column immediately upon creation of Bismuth-213 after decay of its precursor. As mentioned above, the eluant flow may contain a flow of active targeting moiety molecules that reflects the overdose needed to obtain the predetermined ratio in view of the number of Bismuth-213 released from the Actinium-225 per time unit from its source. In another embodiment, the eluent flow with targeting moieties is set to at least capture all Bismuth-213 released from the Actinium-225 per time unit from its source, and the eluted composition is diluted with cold targeting moieties to arrive at the predetermined ratio prior to injection into the patient.

The targeting moiety may comprise an antibody, or antibody fragment that binds to a cell-surface antigen. The pathological site may comprise diseased cells, e.g. tumor cells, cells at an inappropriate site, e.g. neutrophils at a site of inflammation, platelets at sites of injury, or it may be an extracellular structure such as fibrin or thrombin within a coronary vessel at a site of infarct, cholesterol at a site of atherosclerotic plaque, calcification in diseased vessels, amyloid plaque at diseased sites in the brain, or the like.

As noted above, the targeting moiety of the radioconjugate and the target moiety of the pathological site constitute a specific binding pair. The expression "specific binding pair" as used herein refers to complementary substances that selectively recognize and interact with each other to the substantial exclusion of other substances. Representative examples of specific binding pairs include, without limitation, antibodies-antigens (which may be monoclonal or polyclonal immunoglobulins or immunoreactive fragments thereof).

The targeting moiety may preferably be a monoclonal antibody or a fragment or a derivative thereof. Preferably such an antibody is a human or a humanized antibody to prevent immunological reactions.

Of course fragments and/or derivatives of the targeting moieties can also be used as long as they retain a substantial amount of target specificity. Thus for this invention it should be understood that where a targeting moiety is mentioned, one should also consider a fragment or a derivative thereof, as well as any other appropriate site-selective moiety as part of the invention.

Preferably antibodies are directed against tumor associated antigens, such as CEA (carcino-embrionic antigen), AFP (alpha-foetoprotein) PHAP (fast homoarginine-sensitive alkaline phosphatase), p97 (melanoma specific) and EL-1 (elongation factor 1), Antibody B3 and HuM195 (e.g. from Memorial Sloan-Kettering Cancer Center in New York City). Suitable antibodies may be obtained with the method described in EP 0151030.

The coupling of the Bismuth-213 to the targeting moiety can be done in any suitable way as long as the targeting specificity of the targeting moiety is not hampered to a substantial amount.

Preferably the coupling will be done through one of the now many known chelating agents. Suitable chelating agents are DTPA (diethylenetriaminepenta acetic acid) and its derivatives, PLED or its derivatives, EDTA or its derivatives or crownethers or its derivatives. Usually a chelate such as for instance DTPA is bonded to the targeting moiety by means of an organic functional group. It is important to have the Bismuth-213 securely coupled to the chelating agent and thus the targeting moiety. These techniques are known in the art and need no further elucidation here.

The invention also provides for a conjugate composition as produced by the method of the invention as well as a pharmaceutical formulation comprising such a conjugate composition and a pharmaceutically acceptable medium for injection. Suitable pharmaceutically acceptable media for injection may include physiological saline solution, solvents, dispersion media, antibacterial and antifungal agents etcetera.

A method is provided for producing the conjugate composition comprising a targeting moiety and a radioisotope and administering it to the patient without delay or any necessary actions of the therapist. The conjugate composition according to the invention can also be used in combination with a pretargeting moiety. In that case the pretargeting moiety is administered to the patient beforehand so that it can reside at the diseased cells. Since in that case the target site is defined by the pretargetting moiety present at the diseased cells, the predeterminent ratio must be determined with respect to the amount of pretargeting moiety present per diseased cell.

As mentioned-above, intermittent processing is possible with the conjugate composition according to the invention. However, this intermittent processing is subject to loss of isotopes during the process as explained below. The maximum dosage size of each successive step, by the nature of the radioactive decay laws, is subjective to the size, in millicuries, of the ²²⁵Ac-source minus the various losses in time and in operations on the way to the actual administration of the drug

With the apparatus according to the invention, as for instance depicted in Figure 1, this loss of isotopes during processing can be avoided, because it provides for continuous loading of the targeting moiety and subsequently continuous administration to the patient. Also, the hold-up of the mixing vessel 7 will deminish eventual dose rate variations.

For continuous loading the targeting moiety and administration of the formed pharmaceutical formulation, the source material, ²²⁵Ac is provided in a choice of standard amounts (A₀) of 1, 2, 5, 10, 20 or 50 milli Curies, of medically approved purity, and supplied to the hospitals by a central ²²⁵Ac production plant. Said production plant provides the Actinium-225 in the form of ²²⁵Ac₂O₃ or ²²⁵AcCl₃ on an ion exchange resin in a column depicted as 4 in figure 1. The ion exchange resin 14 can be selected from a variety of various well known materials for the purpose, like AGMP-50 resin (BioRad Labs, Hercules, CA), Zr(Phosponate)ₓs (Sylvester, et.al, Lyntech,Inc, Coll.Station,TX, US pat. 7,211,231 B2) or Dowex-50 ion exchanger (Sigma Aldrich).

The elution liquid 11, a solution of the chelator-containing targeting moïety in, for example, diluted, buffered hydroclorid acid of pH 5 - 6, prepared by the hospital laboratory, is stored in vessel 1 and, when operating, will be administered by a standard 2-or 3-channel medical peristaltic infusion pump **6** constantly in amounts of, for example 2 ml/min.

The radioconjugate composition of the invention may be used not only for treating cancer, immune or infectious diseases, but also for the treatment of inflammatory conditions, such as rheumatoid arthritis, arteritis, endometritis or the like, as well as for the removal of new growth or other abnormal tissue. These without limitation, fibrin or thrombin within a coronary vessel at the site of infarct, cholesterol at the site of atherosclerotic plaque, calcification in diseased vessels, amyloid plaque at diseased sites in the brain, or the like. For the purpose of this invention all this tissue to be treated are referred to as diseased cells.

The present invention is further illustrated by means of several examples and figures. These are merely meant for illustration and should not be considered as limitative. Elements and aspects discussed for or in relation with a particular embodiment may be suitably combined with elements and aspects of other embodiments, unless explicitly stated otherwise.

### EXAMPLES AND DESCRIPTION OF THE DRAWINGS

It is noted that the drawings are schematic, not necessarily to scale and that details that are not required for understanding the present invention may have been omitted.

Further, elements that are at least substantially identical or that perform an at least substantially identical function are denoted by the same numeral.
Fig. 1 schematically depicts an embodiment of an apparatus A for practizing the invention as described herein; the apparatus A comprises a first vessel 1, a second vessel 2, an ion exchange column 3, and patient catheter 4 for administration of a liquid to a patient P. The vessels 1, 2, the column 3 and the catheter 4 are connected with respective conduits 51, 52, 53. The conduits 51, 52, 53 may be provided with valves and/or pumps 62, 63, at least some of which may be part of one device, e.g. a multi-channel peristaltic infusion pump (as generally indicated with a dashed line and identified with reference number 6). The apparatus A further comprises a mixing chamber 7.
The first vessel 1 contains an eluent 11 and chelator-containing targeting moiety. The second vessel 2 contains an infusion liquid 12. The ion exchange column 3 contains a bound parent radiometal 13 (²²⁵Ac).
In use, an eluent 11 comprising targeting moiety passes from vessel 1 through an ion exchange column 3 where a daughter radioisotope (²¹³Bi) is stripped from the source isotope 13 (²²⁵Ac) located in that column 3. The ²¹³Bi in the eluent then becomes bound to the targeting moiety and elutes with the eluent.. The resulting fluid is mixed with infusion fluid 12 from vessel 2 and administered to the patient P, for which a mixing vessel 7 is provided. The apparatus is driven by a 3-channel medical peristaltic infusion pump 6. Also or alternatively, the apparatus can be installed on and operated from a vertical equipment pole adjacent to the side of the bed of the patient. Optionally, the liquid 12 from vessel 2 contains a certain overdose of "cold" targeting agent, i.e. the monoclonal antibody (HuM195) without the chelator moiety for diluting the conjugate to the predetermined ratio of cold and loaded targeting moieties.

### EXAMPLE 1

Due to its halflife of 10,2 days, a source of 2 milliCuries of ²²⁵Ac will loose 50 % of its activity in ≈10 days. So, after the first ten days when it lost ≈ 1 mCi it has still 1 mCi left. Because the decay rate has a logarithmic relation with elapsed time, during the first 5 days the ²²⁵Ac source lost 69 % of its activity at day 10. So, after the first 5 days it has lost 35% of its starting activity of 2 mCi, leaving only (1-0,35)x2 ≈ 1,3 mCi ²²⁵Ac. The same reasoning goes for ²¹³Bi because, due to its relatively short halflive of 48 minutes, for all practical purposes its activity follows the decay rate of ²²⁵Ac with time.
During the first day of operation the ²²⁵Ac source lost ≈ 10% of its activity. The same happened to the amount of ²¹³Bi that could be extracted from the original source of 2 mCi ²²⁵Ac. In terms of ²¹³Bi this would amount to 10% of 2 x t_{½,Ac}/t_{½,Bi} =0,1 x 2 x 10 x 24 x 60/48 mCi/day = 0,2 x 300 mCi/day = 60 mCi/24 h = 2,5 mCi/h of ²¹³Bi. Counting with 20% operatng loss, the net amount of ²¹³Bi raised at the start of operation then would be 2 mCi/h for an actinium source of 2 mCi ²²⁵Ac. During 24 h of continuous operation a dose of ≈ 50 mCi of ²¹³Bi bound drug will be administered to the patient. That is the amount needed to completely eliminate 1 kg of AML-tumor from the blood of the patient.

### Example 2 (separate addition of cold antibodies)

A patient is treated using the apparatus according to figure 1. In total an overdose of 1000 X HuM195 with respect to the bismuth-213 formed, is used during operation. During operation an overdose of 100 X HuM195 with the chelator moiety in diluted, buffered hydroclorid acid of pH 5 - 6, stored in vessel 1, is passed through the column 3, the rest of the HuM195 without chelator is added together with injection fluid from vessel 2. The resulting pharmaceutical formulation is administered by a standard 2-channel medical peristaltic infusion pump 6 constantly in amounts of, for example 2 ml/min. During 24 h of continuous operation through the apparatus according to figure 2 a dose of ≈ 50 mCi of ²¹³Bi bound drug will be administered to the patient. That is the amount needed to completely eliminate 1 kg of AML-tumor from the blood of the patient.

### EXAMPLE 3 (comparative):

### The use of equimolar amounts of moieties in batchwise processing)

With batch-wise processing the ²¹³Bi separated from the ²²⁵Ac-source by elution are contacted with a solution of the targeting moïety onto which the chelating agent previously has been added. A 15 mCi ²¹³Bi sample being reacted with an about equal amount of chelated targeting moïety (on a gmol basis) will lead *at best* to a 95% binding of the ²¹³Bi after 15 minutes reaction time. The remaining, free ²¹³Bi needs to be removed and is lost.

## Claims

1. Method for the preparation of a radiolabled conjugate composition for treating diseased cells comprising targeting moieties and Bismuth-213 atoms wherein:
- The conjugate composition comprising Bismuth-213 loaded targeting moieties that have been diluted with cold targeting moieties in a predetermined ratio of amount of cold targeting moieties to amount of Bismuth-213 loaded targeting moieties,
- Said ratio is determined by:
a. Determining the amount T of target moieties per diseased cell to be treated, and
b. Determining the amount Z of Bismuth-213 atoms needed to kill a diseased cell, and
c. Contacting targeting moieties with Bismuth-213 atoms in a ratio of T over Z,
to form a conjugate composition with a predetermined ratio of cold targeting moieties and Bismuth-213 loaded targeting moieties.

2. Method according to claim 1 wherein step c is performed by guiding a stream of eluent comprising targeting moieties over an appropriate medium that is loaded with Actinium-225 for a period of time so that any Bismuth-213 formed is coupled to a targeting moiety.

3. Method according to claim 2 wherein the stream of eluent is guided over the medium with a speed that is set to obtain a composition with the predetermined ratio in amount of cold targeting moieties and Bismuth-213 loaded targeting moieties.

4. A method according to any one of preceding claims, **characterized in that** the targeting moiety is a monoclonal antibody, or a fragment or a derivative thereof.

5. A method according to any one of preceding claims, **characterized in that** the targeting moiety is a human or humanized antibody, or a fragment or a derivative thereof.

6. A method according to claim 5, **characterized in that** the monoclonal antibody is directed to a complementary antibody of a tumor associated antigen.

7. A method according to anyone of the aforegoing claims 1-6, **characterized in that** the targeting moiety is coupled to the Bismuth-213 by means of a chelating agent.

8. A method according to anyone of the aforegoing claims 1-9, **characterized in that** a carrier is coupled to the targeting moiety, whereby said carrier becomes loaded with radioisotopes out of said appropriate medium.

9. A conjugate composition comprising a targeting moiety and a radioisotope, obtainable by any of methods according to claims 1-9.

10. A pharmaceutical formulation, **characterized in that** it comprises a conjugate composition according to claim 10 and a pharmaceutically acceptable medium for injection.
